# EUROPEAN PATENT APPLICATION

(11) **EP 1 138 327 A1**
(43) Date of publication of application: **04.10.2001**
(21) Application number: 00400868.6
(22) Date of filing: 29.03.2000
(51) Int. Cl.: A61K 31/40, A61K 31/445, A61K 31/55, A61P 1/00

(54) **Use of 2-(arylalkenyl)azacycloalkanes derivatives for treating stress-related gastrointestinal disorders**

(71) Applicant: WARNER-LAMBERT COMPANY, Morris Plains New Jersey 07950 (US)
(72) Inventor: Bueno, Lionel, 31840 Aussonne (FR); Chovet, Maria, 92120 Montrouge (FR); Roman, François, 94400 Vitry sur Seine (FR)
(74) Representative: Cabinet Hirsch

(57) **Abstract**

The invention relates to the use of 2-(arylalkenyl)azacycloalkane derivatives, and particularly of (S)-1-cyclopropylmethyl-2-[(E)-3-(3,4-dichloro-phenyl)-allyl]-piperidine, for the prevention and the treatment of stress-related gastrointestinal disorders.

## Description

### Field of the invention

The invention relates to the use of 2-(arylalkenyl)azacycloalkane derivatives, and particularly of (S)-1-cyclopropylmethyl-2-[(E)-3-(3,4-dichloro-phenyl)-allyl]-piperidine, for the prevention and the treatment of stress-related gastrointestinal disorders.

### Background of the invention

Stress is a factor known to increase susceptibility to gastrointestinal disorders and to exacerbate symptoms related to these pathologies.

It has been shown that stress is an exacerbating factor in gastrointestinal disorders such as functional bowel disorders and inflammatory bowel diseases. But stress is also a triggering factor in these pathologies, as demonstrated in post-infectious irritable bowel syndrome (Gwee and al., Gut, 1999, 44:400-406). Sufferers of these diseases generally require greater medical intervention when stress is a continuing additional factor than sufferers where this factor has been alleviated.

### Summary of the invention

The invention relates to the use of 2-(arylalkenyl)azacycloalkanes of formula (I), 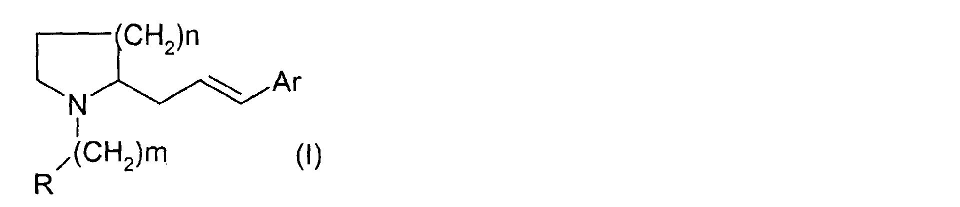
in which Ar is aryl or heteroaryl, optionally mono- to trisubstituted,
m has the value 1 or 2,
R is phenyl, optionally substituted, or cycloalkyl containing 3 to 7 carbon atoms,
n has the value 1 to 3,
or a pharmaceutically acceptable derivative thereof for the preparation of a medicament for the prevention and the treatment of stress-related gastrointestinal disorders.

The invention relates to a method for treating a patient suffering from a stress-related gastrointestinal disorder, comprising administering an effective amount of the above compound to said patient.

The invention is disclosed in more details in the following specification, and in reference to the drawings, in which:
Figure 1 shows the effect of (S)-1-cyclopropylmethyl-2-[(E)-3-(3,4-dichloro-phenyl)-allyl]-piperidine on stress-induced hypersensitivity to rectal distension;
Figure 2 shows the effect of (S)-1-cyclopropylmethyl-2-[(E)-3-(3,4-dichloro-phenyl)-allyl]-piperidine on stress-induced colonic hyperkinesia.

### Detailed description of the invention

The invention relates to the novel use of the compound of formula (I) : 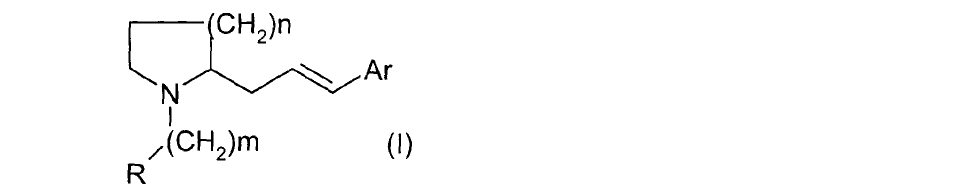
in which Ar is aryl or heteroaryl, optionally mono- to trisubstituted,
m has the value 1 or 2,
R is phenyl, optionally substituted, or cycloalkyl containing 3 to 7 carbon atoms,
n has the value 1 to 3,
their isomers and their addition salts in the treament of stress-related gastrointestinal disorders.
- The term aryl means unsaturated mono- or bicyclic carbon-containing radicals such as phenyl, indenyl or naphthyl, which may be partially saturated as indanyl or tetrahydronaphthyl. The phenyl radical, optionally mono- to trisubstituted, is preferred. In this case, the substituents, which may be identical or different in the case of multiple substitution, are preferably selected from the group consisting of halogens, the nitro group, C1 to C4 alkyl, haloalkyl, alkoxy and haloalkoxy radicals.
- The term heteroaryl means unsaturated mono- or bicyclic radicals comprising one or two heteroatoms preferably selected from oxygen, nitrogen and sulfur.
- The term halogen means chlorine, bromine or fluorine.

Preferred compounds of the invention are those in which,
- Ar is a phenyl radical, mono-, di- or trisubstituted by halogen atoms,
- R is cyclopropyl, cyclobutyl or phenyl,
- n has the value 1 or 2, and ,
- m has the value 1 or 2.

The preparation of compounds of formula (I) is disclosed in WO-A-9515948, the content of which is incorporated herein by reference

The most preferred compound to be used in the context of the invention is (S)-1-cyclopropylmethyl-2-[(E)-3-(3,4-dichloro-phenyl)-allyl]-piperidine, which has the following formula (A) :

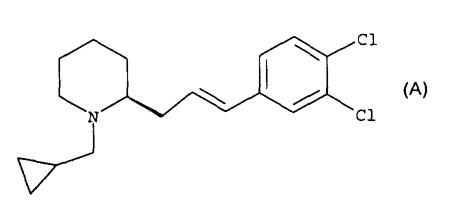

The preparation of this compound is disclosed in WO-A-9839296, the content of which is incorporated herein by reference.

The compounds utilized in the invention include solvates, hydrates, pharmaceutically acceptable salts, and polymorphs (different crystalline lattice descriptors) of the compounds of Formula I, which are pharmaceutically acceptable derivatives thereof.

Where it is appropriate to form a salt, the pharmaceutically acceptable salts include acetate, benzenesulfonate, benzoate, bicarbonate, bitartrate, bromide, calcium acetate, camsylate, carbonate, chloride, citrate, dihydrochloride, edetate, edisylate, estolate, esylate, fumarate, gluceptate, gluconate, glutamate, glycoloylarsanilate, hexylresorcinate, hydrabamine, hydrobromide, hydrochloride, hydroxynaphthoate, iodide, isethionate, lactate, lactobionate, malate, maleate, mandelate, mesylate, methylbromide, methylnitrate, methylsulfate, mucate, napsylate, nitrate, pamoate (embonate), pantothenate, phosphate/diphosphate, polygalacturonate, salicylate, stearate, subacetate, succinate, sulfate, tannate, tartrate, theoclate, triethiodide, benzathine, chloroprocaine, choline, diethanolamine, ethylenediamine, meglumine, procaine, aluminum, calcium, lithium, magnesium, potassium, sodium, and zinc. Further teachings are shown by Berge S.M. and *al*. (1977), "Pharmacological salts", J. Pharm. Sci. 66: 1-19, the contents of which is incorporated herein by reference.

Use of a prodrug of a compound of the invention, such as would occur to one skilled in the art (see Bundgaard, et *al.,* Acta Pharm. Suec., 1987; 24: 233-246), is also contemplated.

Compounds of formula I are usually provided as a pharmaceutical composition comprising the compound together with at least one pharmaceutically acceptable carrier or excipient. For preparing pharmaceutical compositions from the compounds used in this invention, inert, pharmaceutically acceptable carriers can be either solid or liquid.

Solid form preparations include powders, tablets, dispersible granules, capsules, sachets, and suppositories. A solid carrier can be one or more substances which may also act as diluents, flavoring agents, solubilizers, lubricants, suspending agents, binders, or tablet disintegrating agents; it can also be an encapsulating material. In powders, the carrier is a finely divided solid, which is in a mixture with the finely divided active component. In tablets, the active component is mixed with the carrier having the necessary binding properties in suitable proportions and compacted in the shape and size desired. For preparing suppository preparations, a low-melting wax such as a mixture of fatty acid glycerides and cocoa butter is first melted and the active ingredient is dispersed therein by, for example, stirring. The molten homogeneous mixture is then placed in convenient sized molds and allowed to cool and solidify. The powders, tablets, sachets or encapsulated forms for capsules preferably contain 5% to about 70% of the active component. Suitable carriers include but are not limited to magnesium carbonate, magnesium stearate, talc, lactose, sugar, pectin, dextrin, starch, tragacanth, methyl cellulose, sodium carboxymethyl cellulose, a low-melting wax, cocoa butter, and the like.

Tablets, powders, sachets, and capsules can be used as solid dosage forms suitable for oral administration.

Liquid form preparations include solutions, suspensions, and emulsions.

Sterile water or water-propylene glycol solutions of the compounds of formula (I) are an example of liquid preparations suitable for parenteral administration. Liquid preparations can also be formulated in solution in aqueous polyethylene glycol solution.

Aqueous solutions for oral administration can be prepared by dissolving the active component in water and adding suitable colorants, flavoring agents, stabilizers, and thickening agents as desired. Aqueous suspensions for oral use can be made by dispersing the finely divided active component in water together with a viscous material such as natural synthetic gums, resins, methyl cellulose, sodium carboxymethyl cellulose, and other suspending agents known in the pharmaceutical formulation art.

Preferably the pharmaceutical preparation is in unit dosage form. In such form, the preparation is divided into unit doses containing appropriate quantities of the active component. The unit dosage form can be a packaged preparation, the package containing discrete quantities of the preparation, for example, packeted tablets, capsules, and powders in vials, ampoules or sachets. The unit dosage form can also be a capsule, a sachet or a tablet itself, or it can be the appropriate number of any of these packaged forms or combination of the above.

The dosage format will be such that the amount of the compound of formula (I) administered will generally be from about 0,1 mg to about 400 mg per day. Preferred doses will be from about 0,1 mg to about 100 mg per day for an adult of 70 kg.

The invention therefore concerns the use of a compound of formula (I) for preventing and treating stress-related gastrointestinal disorders. Such disorders include, among other conditions, stress-induced functional bowel disorders and stress-induced aggravation of inflammation in inflammatory bowel diseases.

Stress-induced functional bowel disorders comprise stress-induced irritable bowel syndrome (IBS), stress-induced functional bloating and stress-induced functional abdominal pain.

Stress-induced IBS comprises post-infectious IBS and symptoms related to IBS. These symptoms comprise altered bowel habits such as abdominal pain, bowel distension, intestinal bloating, constipation and colonic motility disorders.

Stress-related inflammatory bowel diseases comprise colitis, ulcerative colitis and Crohn's disease.

The forms of stress able to induce gastrointestinal disorders or exacerbation of symptoms related to these pathologies include emotional stress, exam-induced stress, severe stress, anxiety and all others kinds of stresses.

The invention can be applied to any mammal, including humans.

The invention also provides a method for the prevention and the treatment of stress-related gastrointestinal disorders comprising administering to a mammal, preferably a human, in need thereof, an effective amount of a compound of formula I.

The following examples illustrate the invention without limiting it.

### Example 1. Stress induced rectal hypersensitivity

### 1.1 Animal preparation

Individually housed 250-300g female Wistar rats (Janvier SA, Le Genest St. Isle, France) were used. The animals first were premedicated with acepromazine (Camivet, Vetiquinol, Lure, France, 0.5 mg/kg) and Ketamine (Imalgene, Rhone Merieux, Toulouse, France, 100 mg/kg) injected intraperitoneally. Animals were prepared for electromyographic recordings. Pairs of nickel/chrome wire electrodes were implanted in the striated muscle of the abdomen, 2 cm laterally of the white line. The free end of the electrodes were exteriorized on the back of the neck and protected by a plastic tube attached to the skin.

### 1.2 Electromyographic recordings

Electromyographic recordings were commenced 8 days post surgery. Bipolar recordings of myoelectric activity were performed with an electroencephalographic recorder (Mini VIII Alvar, Paris, France) using a short time constant (0.03 s) to suppress low-frequency signals (< 3 Hz) and a paper speed of 3.6 cm/min. Spike bursts were recorded as an index of abdominal contractions.

### 1.3 Stress procedure

Partial restraint stress, a relatively mild non-ulcerogenic model of restraint, was used in all stress sessions. Animals were lightly anaesthetized with ethylether, and their fore-shoulders, upper forelimbs and thoracic trunk were wrapped in a confining harness of paper tape to restrict, but not to prevent their body movements. Then animals were placed in their home cage for 2 hours. The rats recovered from anaesthesia within 2-3 min and immediately moved about in their cages. Partial restraint stress was always performed between 10:00 and 12:00 a.m.

Compound A was administered by subcutaneous route at dosages of 2, 10 or 50 *µ*g/kg, 30 minutes before the stress session.

### 1.4 Rectal distension procedure

In order to prevent recording artefacts during the distension period, rats were placed in plastic tunnels (6 cm diameter x 25 cm long), where they were acclimated 3 days before rectal distension (RD). The balloon used for RD was an arterial embolectomy catheter (Fogarty, Edwards Laboratories, Inc). RD was performed by insertion of the balloon (2 mm diameter x 2 cm long) into the rectum, at 1 cm from the anus, and the catheter was fixed at the base of the tail. The Fogarty probe was inflated progressively with tepid water in steps of 0.4 ml, from 0 to 1.2 ml, each step of inflation lasting 5 min. To detect possible leakage, the volume of water introduced in the balloon was checked by complete removal with a syringe at the end of the distension period.

Rectal distension was performed immediatly after the stress session.

### 1.5 Results

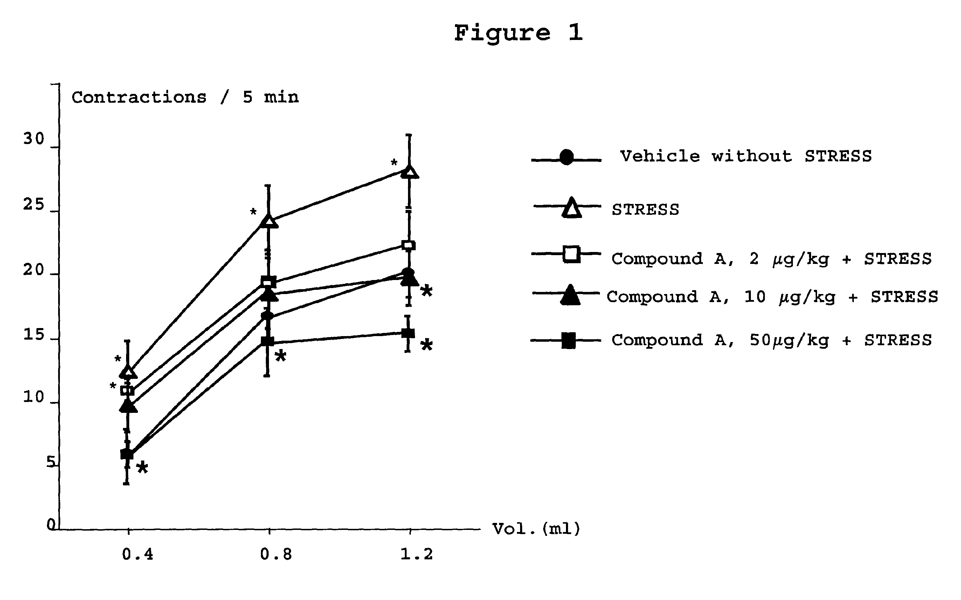

Figure 1 shows the number of abdominal contractions per period of 5 minutes, as a function of the volume of water introduced in the Fogarty probe. The * sign indicate that p<0.05 which indicates that the result obtained is significantly different from vehicle values. The sign * indicates that p<0.05 which indicates that the result obtained is significantly different from stress values. The term vehicle means the injected solution without compound A.

As shown in the figure, the compound of formula A reduces (at 2 *µ*g/kg) or suppresses (at 10 and 50 *µ*g/kg) stress-induced hypersensitivity to rectal distension in awake rats.

### Example 2. Lipopolysaccharide (LPS) induced rectal hypersensitivity in rats

### 2.1. Animal preparation

Rats were anaesthetized by intraperitoneal injection of acepromazine (0.6mg/kg, Calmivet, Vetiquinol, Lure, France) and ketamine (120mg/kg, Imalgene 1000, Rhone Merieux, Lyon, France). Three groups of three electrodes were implanted in the abdominal external oblique musculature, just superior to the inguinal ligament. Electrodes were exteriorized on the back of the neck and protected by a glass tube attached to the skin. Animal were individually housed in polypropylene cages and kept in a temperature-controlled room (21°C). Food (UAR pellets, Epinay, France) and water were provided *ad libitum.*

### 2.2. Electromyographic recording

Electromyographic recordings were commenced 5 days post surgery. The electrical activity of abdominal striated muscles was recorded with an electroencephalographic recorder (Mini VIII Alvar, Paris, France) using a short time constant (0.03 s) to supress low-frequency signals (< 3 Hz) and a paper speed of 3.6 cm/min. Spike bursts were recorded as an index of abdominal contractions.

### 2.3.Rectal distension procedure

In order to prevent recording artefacts during the distension period, rats were placed in plastic tunnels (6 cm diameter x 25 cm long), where they were acclimated 4 days before rectal distension (RD). The balloon used for RD was an arterial embolectomy catheter (Fogarty, Edwards Laboratories, Inc). RD was performed by insertion of the balloon (2 mm diameter x 2 cm long) into the rectum, at 1 cm from the anus, and catheter was fixed at the base of the tail. The Fogarty probe was inflated progressively with tepid water by steps of 0.4 ml, from 0 to 1.2 ml, each step of inflation lasting 5 min. To detect possible leakage, the volume of water introduced in the balloon was checked by complete removal with a syringe at the end of distension period.

### 2.4. Experimental protocol

Rats were injected by the intraperitoneal route with LPS (Escherichia coli, serotype 0111:B4) (1mg/kg) or its vehicle, and rectal distension with concomitant electromyographic recording of abdominal contractions was performed 9 and 12 hours after this administration. To determine the antinociceptive properties of the compound of formula A in hyperalgesia conditions, compound A or the vehicle (NaCl 0.9% 0.3ml/rat) were administered by subcutaneous route at 30 min before rectal distension and 12 hours after LPS (or its vehicle) injection.

### 2.5. Results

Compound A administered at 50 µg/kg by subcutaneous route, did not produce any significant activity on LPS-induced rectal hypersensitivity.

### Example 3. Emotional stress and colonic motility

### 3.1 Animal preparation

Male Wistar rats (Centre d'élevage R. Janvier, Route des Chenes Secs, 53940 Le Genest Saint Isle, France), weighing 250-300 g, were used for these experiments. Animals were housed individually in polypropylene cages (37.5 x 17 x 15 cm) kept in a temperature controlled room (21 ± 1°C, 50 ± 5% relative humidity) on a 12-h light-dark cycle (light on at 8:00 a.m.). The rats were allowed access to water *ad libitum* and rat chow between 8:00 and 12:00 p.m.

### 3.2 Motility recordings

Ketamine (Imalgene 1000, Rhône Merieux, Lyon, France), (100 mg/kg i.p.) anaesthetized animals were surgically prepared with nickel/chrome electrodes implanted into the muscular layers of the proximal colon at 2 and 4 cm distal from the ileo-colonic junction according to a previously described method (Ruckebusch and Fioramonti, 1975, Gastroenterology, 68:1500-1508). The animals were also fitted with a permanent polyethylene catheter (PE 10) inserted into the lateral ventricle of the brain (L: 1.8; A: -1.3; H: -3.5) according to the atlas of Paxinos and Watson (1986). Bipolar recordings of myoelectric activity (time constant, 0.01 s) using an electromyographic recorder (Minihuit, Alvar, Paris, France) began 5 days after surgery. The rats were fasted for 12 h before each experiment and colonic myolectric activity was recorded from 30 min before to 60 min after the emotional stress session.

### 3.3 Emotional stress session procedure

The emotional stress involved displacement of the animals from their cage to a closed box (test-cage) in which they had previously received electric shocks as previously described (Gué et al., 1991, "Conditioned emotional response in rat enhances colonic motility through the central release of CRF", Gastroenterology 100, 964). Briefly, each rat was placed in the test-cage for 5 min and received six series of electric footshocks (1.4 mA-180 ms) supplied to the grid floor by a pulse generated scrambler (Campden® England). Each series of shocks lasted 8 s and was administered at 45-s intervals. As the resistance, with the animal in the cage, varied between 10 and 250 kQ, each rat received an electric footshock in the range of 1.2 to 3.0 mA, a value producing discomfort but not pain as judged by the lack of any jump response. Placement of the rat in the same cage, without electric shock was considered to be a form of emotional stress (Nabeshima et al., 1988, "Inhibition of enkephalin degradation attenuated stress-induced motor suppression (conditioned suppression of motility)", J. Pharmacol. Exp. Ther. 244, 303; Gué et al., 1991). In order to eliminate the ability of rats to differentiate the electric footshocks session from the emotional stress session without electric footshocks, reinforcement was performed at 2-day intervals with each rat in a randomized fashion (for details see Gué et al., 1991). The study was approved by the Midi-Pyrénées Experimentation Committee (agreement No. 9125).

### 3.4 Colonic motility evaluation and statistics

Colonic spike bursts frequency, expressed as the number of spike bursts occurring in 10-min periods, was determined by analysis of electromyographic recordings, 30 min before or after the beginning of each stress session. The mean values obtained before and during emotional stress as well as their differences were compared using Student's paired t-test or two way analysis of variance (ANOVA) followed by a paired t-test for each treatment. Differences were considered to be significant for P < 0.05.

### 3.5 Results

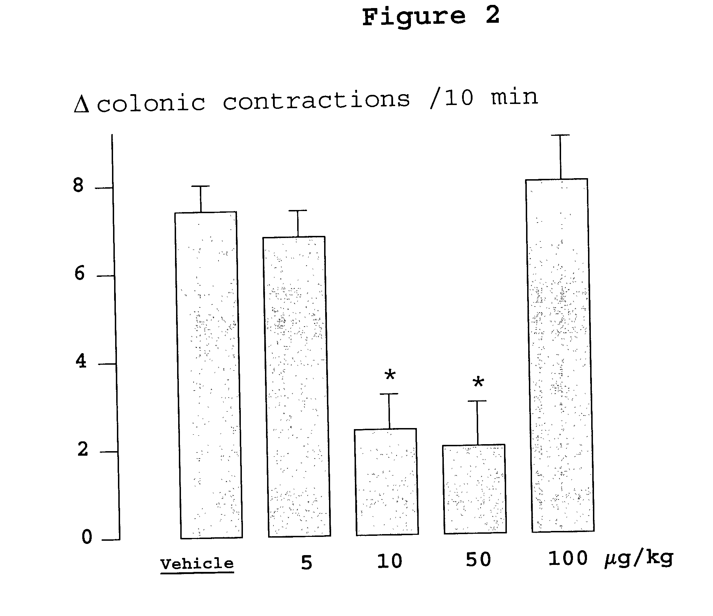

Figure 2 shows the number of colonic contractions occuring in 10 minutes-period as a function of the product injected (vehicle or compound A at different concentrations). The * sign indicate that p<0.05 which indicates that the result obtained is significantly different from vehicle values. The term vehicle means the injected solution without compound A.

After treatment by subcutaneous route 20 min before the emotional stress session, the following results were obtained : At 10 and 50 *µ*g/kg the compound of Formula A strongly and significantly (P<0.05) reduces the increase in the frequency of colonic contractions associated with emotional stress in awake rats , as indicated in the figure.

### Example 4. Stress-induced reactivation of colitis in rats

### 4.1 Subjects

Male Wistar rats (Centre d'élevage R. Janvier, Route des Chênes Secs, 53940 Le Genest Saint Isle, France), weighing 250-300 g, are used for these experiments. Animals are housed individually in polypropylene cages (37.5 x 17 x 15 cm) kept in a temperature controlled room (21 ± 1°C, 50 ± 5% relative humidity) on a 12-h light-dark cycle (light on at 8:00 a.m.). Water and food are available *ad libitum*.

### 4.2 Induction of colitis

TNBS is dissolved in 50% ethanol to a concentration of 120 mg/mL. Rats are anesthetized with ether, and a polyethylene (PE)-60 catheter is inserted 8 cm past the anus. Rats are infused with 0.25 ml of the TNBS solution at a dose of 30 mg TNBS/mL.

### 4.3 Stress procedure

Partial restraint stress, a relatively mild, non-ulcerogenic model of stress, is used in all stress sessions. The foreshoulders, upper forelimbs and thoracic trunk of the animals are wrapped in a confining harness of paper tape. This restricts but does not prevent their body movements; then the animals are placed in their home cages for 3 hours. This procedure is repeated once a day during 3 days. The first stress session is performed at the 42nd day post colitis induction.

### 4.4 Assessment of colonic inflammation.

The severity of the colitis is assessed in three ways: macroscopic scoring, histological evaluation, and quantification of granulocyte infiltration through measurement of myeloperoxidase (MPO) activity.

Rats of the randomized treated groups are weighed and sacrificed by cervical dislocation, and the 10 cm of distal colon are removed. The colon is opened by a longitudinal incision, rinsed with saline, and pinned out on a wax block. The macroscopic scoring of colonic damage is performed using criteria which take into consideration the area of damage involvement and the presence or absence of ulcers. The presence or absence of adhesions between the colon and other organs is also noted. Scoring of damage and excision of tissue samples are performed by an observer unaware of the treatment group.

The distal portion of the colon (6-7 cm proximal to anus) is excised in two pieces for histological score and MPO assessment. Thin sections of one piece of the colon (5 *µ*m) are mounted on glass slides and stained with hematoxylin and eosin to reveal structural features. Histological assessment is performed on coded slides to prevent observer bias. The other piece of tissue sample is frozen on dry ice and stored at -80°C for subsequent measurement of MPO activity. MPO activity is measured by the modified technique of Bradley et al., 1982, "Measurement of cutaneous inflammation: estimation of neutrophil content with an enzyme marker", J. Invest. Dermatol.78:206-209. The MPO assays are performed in a blinded fashion on coded tubes. Protein concentration is measured by the modified method of Lowry et al., 1951, "protein measurement with the Folin phenol reagent".J. Biol. Chem. 193:265-275., using the Bio-Rad DC test, and results are expressed as units of MPO assay per gram of tissue protein.

### 4.5 Experimental procedure

Three groups of animals are used and treated with intracolonic TNBS. Two groups undergo the stress procedure and receive either compound A or its vehicle. Compound A or its vehicle is administered by oral route once a day, 1 hour before each stress session. At the end of the third stress session, animals are sacrificed to assess the inflammation score as described above (macroscopic scoring, histological evaluation and MPO activity determination).

## Claims

1. Use of 2-(arylalkenyl)azacycloalkanes of formula (I) 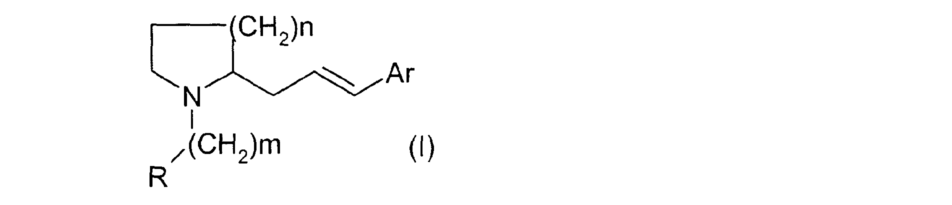
in which Ar is aryl or heteroaryl, optionally mono- to trisubstituted,
m has the value 1 or 2
R is phenyl, optionally substituted, or cycloalkyl containing 3 to 7 carbon atoms,
n has the value 1 to 3,
or a pharmaceutically acceptable derivative thereof, for the preparation of a medicament for the prevention and the treatment of stress-related gastrointestinal disorders.

2. Use according to claim 1, **characterized in that** Ar is a phenyl radical, mono-, di- or trisubstituted by halogen atoms.

3. Use according to claim 1 or 2, **characterized in that** R is cyclopropyl, cyclobutyl or phenyl.

4. Use according to claim 1 to 3, **characterized in that** n and m have independently the value 1 or 2.

5. Use according to claim 1, in which the 2-(arylalkenyl)azacycloalkane is (S)-1-cyclopropylmethyl-2-[(E)-3-(3,4-dichloro-phenyl)-allyl]-piperidine or a pharmaceutically acceptable derivative thereof.

6. Use according to claims 1 to 5, in which the stress-related gastrointestinal disorder is stress-induced functional bowel disorder.

7. Use according to claim 6, in which the stress-induced functional bowel disorder is stress-induced irritable bowel syndrome.

8. Use according to claim 7, in which the stress-induced irritable bowel syndrome is a post-infectious irritable bowel syndrome.

9. Use according to claim 6, in which the stress-induced functional bowel disorder is an altered bowel habit selected from abdominal pain, intestinal bloating, constipation, bowel distensions and colonic motility disorders.

10. Use according to claim 9, in which the stress-induced functional bowel disorder is a colonic motility disorder, functional abdominal pain or bowel distension.

11. Use according to claims 1 to 5, in which the stress-related gastrointestinal disorder is a stress-induced aggravation of inflammation in inflammatory bowel diseases.

12. Use according to claim 11, in which the inflammatory bowel disease is Crohn's disease, colitis or ulcerative colitis.

13. Use according to any one of claims 1 to 12, in which the drug comprises from 0,1 mg to 400 mg of 2-(arylalkenyl)azacycloalkane of formula (I) or a pharmaceutically acceptable derivative thereof.

14. Use according to claim 13, in which the drug comprises from 0,1 mg to 100 mg of 2-(arylalkenyl)azacycloalkane of formula (I) or a pharmaceutically acceptable derivative thereof.

15. Use according to claim 13 or 14, in which the 2-(arylalkenyl)azacycloalkane is (S)-1-cyclopropylmethyl-2-[(E)-3-(3,4-dichloro-phenyl)-allyl]-piperidine or a pharmaceutically acceptable derivative thereof.
